Europäisches Patentamt

European Patent Office

Office européen des brevets

(19)

(11) Publication number: **0 213 724**
**A1**

## (12) EUROPEAN PATENT APPLICATION

(21) Application number: **86305736.0**

(22) Date of filing: **25.07.86**

(51) Int. Cl.⁴ **A61K 31/685 ,**
**//(A61K31/685,31:23)**

---

A request for addition of the symbol on page 17 has been filed pursuant to Rule 88 EPC. A decision on the request will be taken during the proceedings before the Examining Division (Guidelines for Examination in the EPO, A-V, 2.2).

(30) Priority: **26.07.85 US 759270**

(43) Date of publication of application:
**11.03.87 Bulletin 87/11**

(84) Designated Contracting States:
**AT BE CH DE FR GB IT LI LU NL SE**

(71) Applicant: **YEDA RESEARCH AND DEVELOPMENT COMPANY, LTD.**
**Weizmann Institute of Science P.O. Box 26 Rehovot(IL)**

(72) Inventor: **Shinitzky, Meir**
**46 Sderot Chen**
**Rehobot(IL)**

(74) Representative: **Eyles, Christopher Thomas**
**BATCHELLOR, KIRK & EYLES 2 Pear Tree Court Farringdon Road**
**London, EC1R 0DS(GB)**

---

(54) A special lipid mixture for membrane fluidization.

(57) A special lipid mixture for membrane fluidization consisting essentially of about seven parts by weight neutral lipids and about three parts by weight phospholipids, the phospholipids consisting essentially of phosphatidyl choline and phosphatidyl ethanolamine, and the neutral lipids consisting essentially of glycerides.

EP 0 213 724 A1

## A SPECIAL LIPID MIXTURE FOR MEMBRANE FLUIDIZATION

BACKGROUND OF THE INVENTION

Field of the Invention

The present invention relates to a composition which increases the membrane fluidity of biological membranes, to methods for preparing the composition and a method for the treatment or prevention of various disorders using the composition.

Description of the Background Art

Many disorders in man and other mammals are associated with decreases in the fluidity (reciprocal of microviscosity-$^{-1}$) of biological membranes. The fluidity of biological membranes is determined by their structure and chemical composition and, in particular, the mole ratio of cholesterol to phospholipids (C/PL), the mole ratio of sphingomyelin to lecithin (S/L) and the degree of unsaturation of the phospholipid acyl chains - (Shinitzky and Henkart, Int. Rev. Cytol., 60: (1979); Cooper, J., Supramol. Struct., 8:413 (1978)).

In many disorders associated with a decrease in membrane fluidity, the pathogenesis involves changes in membrane lipid composition or lipid metabolism (Cooper, N. Eng. J. Med., 297:371 - (1977)). These changes have been correlated in many cases to an increase in membrane lipid microviscosity of various tissues due to an increase in C/PL or S/L or a decrease in the degree of unsaturation of the phospholipid acyl chains or any combination of the three. Lipid peroxidation can also affect the dynamics of cell membrane proteins and consequently the overt physiological functions (Sagai and Ichinose, Life Sci., 27:731 (1980)). The following is a list of some disorders which have been associated with lipid imbalances, all of which are amenable to lipid manipulations:

(1) Aging and senescence (Yamamoto, Lipids, 3:284 (1968); Rivnay et al., Mech. Age. Dev., 10:71 (1979); Heron et al., Proc. Natl. Acad. Sci. USA, 77:7463 (1980); Araki and Rifkind, Life Sci., 26:2223 (1980); Hershkowitz et al., Progress in Brain Research, Elsevier-North Holland (in press); Ronser et al., Adv. Lipid Res., 10 :262 (1972).

(2) Withdrawal symptoms of drug and alcohol addiction (Johnson et al., Mol. Pharmacol., 15:739 (1979); Chin and Goldstein, Science, 196:684 (1979); Littleton and John, J. Pharm. Pharmac., 29:579 (1977); Heron et al., Biochem. Pharmacol., in press (1982)).

(3) Hyperlipidemic disorders such as hypertension, atherosclerosis, gallstones, cirrhosis, and obesity (Montenay et al., Biochem. Biophy. Res. Comm., 100:660 (1981); Cooper, N., Engl. J. Med., 297: 371 (1977); Miettinen et al., Lancet, 2:835 - (1972)).

(4) Sperm infertility (Davis et al., Biochem. Biophys. Acta, 558 :257 (1979); Davis, Proc. Soc. Exp. Biol. Med., 152:257 (1976)).

(5) Impaired immune function such as in aging, obesity and certain cases of allergies - (Rivnay et al., Mech. Age. Dev., 12:119 (1980); Rivnay et al., Mech. Age. Dev., 10:71 (1979)).

It has also been shown that synaptic membrane microviscosity increases as a result of surgical or chemical lesions of specific pathways in the brain (Heron et al., Biochem. Pharmacol., in press (1982)). These findings may apply to other degenerative or organic damages such as Alzheimer's disease, Parkinsonism, Tardive dyskinesia, Huntington's chorea, tremor, ataxia, and epilepsy and certain cases of mental retardation - (Borri et al., Neurology, 17:172 (1967); Hooghwinkel et al., Neurology, 18:408 (1968); Ueno et al., J. Neurol. Sci., 56:89 (1982)), all of which could in principle be treated by lipid manipulation.

It is also generally accepted that certain mental disorders such as mania, depression and - schizophrenia are related to a chemical imbalance in the turnover rate of neutrotransmitters in the brain. There is evidence to suggest that the biogenic amines (dopamine, norephinephrine and serotonin) are primarily involved. The receptors and membrane-bound enzymes concerned with the turnover of these transmitters can be altered by changes in membrane fluidity (Hershkowitz et al., Progress in Brain Research, Elsevier-North Holland, in press; Heron et al., Proc. Natl. Acad. Sci. USA. 77 :7463 (1980); Heron et al., in Receptors and Their Neurotransmitters, eds. Littauer et al., John Wiley, London (1980); Heron et al., Eur. J. Pharmacol., 72:361 (1981)), and therefore also falls into the category of disorders amenable to lipid manipulations.

Modulation of function by lipid manipulations can also be carried out in vitro. This could be applicable to modulation of viral infectivity for use in vaccinations (Pal et al., Biochemistry, 20:530 - (1981), and antigenicity (Shinitzky and Souroujon, Proc. Natl. Acad. Sci., USA, 76:4438 (1979)), which could reduce tissue rejection and facilitate transplantations.

In the past, such disorders were frequently treated by including a high content of lecithin - (phosphatidyl choline) in the patient's diet. However, in most cases the inclusion of lecithin in the diet has not been found to be particularly effective in alleviating symptoms associated with lipid imbalances and in restoring membrane lipid fluidity to normal. Unfortunately, dietary lecithin dosage levels which have had some effectiveness in increasing membrane fluidity often have adverse effects on a patient's stomach.

There remains a need in the art for a specific, particularly active mixture of lipids having effective membrane fluidization capabilities.

## BRIEF DESCRIPTION OF THE DRAWINGS

FIG. 1 is a schematic, enlarged view of a chylomicron-like particle assembly formed when the composition of the invention is dispersed in aqueous solution.

FIG. 2 is a graphic illustration of the effect of various lipid mixtures on membrane fluidization of human erythrocyte membranes.

FIG. 3 is a graphic illustration of the effect of various lipid mixtures on membrane fluidization of human lymphocyte membranes.

FIG. 4 is a graphic illustration of the cholesterol-removing capabilities of various lipid mixtures with mouse thymocytes.

FIG 5 is a graphic illustration of the effect on human lymphocyte membrane fluidization of phosphatidyl choline compared to the composition of the invention.

FIG. 6 is a graphic illustration of the effects of various lipid mixtures on cholesterol removal from human lymphocytes.

FIG. 7 is a graphic illustration of the effects of a diet containing the composition of the invention on the immune response capabilities of rats.

FIG. 8 is a graphic illustration of the effect of a diet containing the composition of the invention on the immune response capabilities of a first human subject.

FIG. 9 is a graphic illustration of the effect of a diet containing the composition of the invention on the immune response capabilities of a second human subject.

FIG. 10 is a graphic illustration of the effect of a diet containing the composition of the invention on a third human subject.

## SUMMARY OF THE INVENTION

In accordance with the present invention, a composition is provided consisting essentially of about seven parts by weight neutral lipids and about three parts by weight phospholipids, wherein the neutral lipids consist essentially of glycerides and the phospholipids consist essentially of phosphatidyl choline and phosphatidyl ethanolamine. The invention also provides a method for preparing the composition, a method for increasing the fluidity of a biological membrane using the composition, a method for reducing membrane cholesterol content using the composition, and a method for the treatment or prevention of conditions which are mediated by membrane lipid imbalance, conditions which are amenable to membrane lipid manipulation, or conditions which result from an increase in membrane microviscosity.

## DESCRIPTION OF THE PREFERRED EMBODIMENTS

The novel composition of the present invention is a special lipid mixture surprisingly effective for increasing the fluidity of biological membranes.

Phosphatidyl choline (lecithin) is a common membrane fluidizer in nature. However, when phosphatidyl choline is introduced into the blood, it forms stable bi-layers or integrates into serum lipoproteins, both of which are slow in affecting cell membrane fluidity. As a result, when phosphatidyl choline alone is introduced into the bloodstream, most of it is degraded by the liver prior to having an opportunity to affect cell membrane fluidity.

It has, however, been discovered that the rate of membrane fluidization by phosphatidyl choline can be markedly increased when phosphatidyl choline is loosely integrated in structures which facilitate the process of lipid exchange between the membrane and the serum. The formation of these structures is optimized by a special lipid mixture according to the present invention.

Without being bound to any particular theory, it is believed that the effectiveness of the present composition is explained by the formation of loosely integrated lipid structures in an aqueous solution (e.g., blood), which are sufficiently stable in vivo to effectively facilitate translocation of membrane cholesterol and possibly insert exogenous phospholipids into cell membranes.

A special lipid mixture according to the present invention consists essentially of neutral lipids (NL) and phospholipids (PL) in about a 7:3 ratio by weight respectively, with the phospholipids consisting essentially of phosphatidyl choline (PC) and phosphatidyl ethanolamine (PE). Advantageously, the phospholipids are present in the special lipid mixture in a ratio of about 2 parts by weight phosphatidyl choline and 1 part by weight phosphatidyl ethanolamine, to provide a lipid mixture consiting essentially of 7:2:1 ratio by weight of neutral lipids, phosphatidyl choline and phosphatidyl ethanolamine, respectively.

Phosphatidyl ethanolamine is a natural phospholipid which has been found to destabilize lipid bi-layers and can disintegrate stable phosphatidyl choline liposomes. However, it has also been found that mixtures of phosphatidyl choline and phosphatidyl ethanolamine alone tend to precipitate in aqueous solutions, and are therefore not particularly useful as membrane fluidizers in vivo.

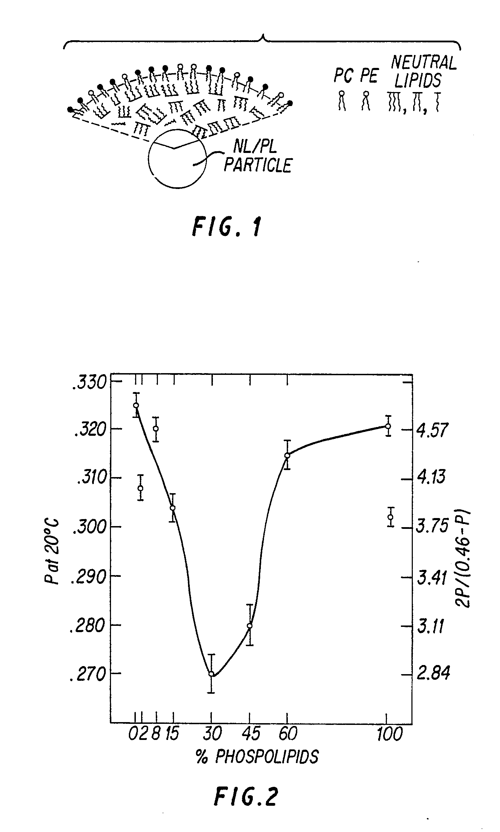

The inventor has, however, found that a stable emulsion in an aqueous solution may be formed with a mixture of phosphatidyl choline, phosphatidyl ethanolamine and neutral lipids consisting essentially of glycerides. A stable emulsion of these lipids which is formed in an aqueous solution is comprised of chylomicron-like assemblies wherein the glycerides serve as a hydrophobic carrier on the surface of which the phosphatidyl choline and phosphatidyl ethanolamine molecules are randomly spread. See Fig. 1.

The specific lipid mixture of the present invention has been found to form these chylomicron-like assemblies in aqueous solutions (e.g., blood) and has been found to be a particularly potent agent for reducing membrane cholesterol content and/or increasing membrane fluidity.

The lipid components included in a composition according to the present invention are preferably derived from natural sources such as egg yolk, soy beans, plant or animal materials, or mixtures thereof, and most preferably are derived from egg yolk.

The neutral lipids utilized in the lipid mixture of the invention consist essentially of glycerides. The neutral lipids component of the mixture may contain small amounts of non-essential lipid impurities which remain associated with the glycerides during isolation of the neutral lipids. These impurities may include, for example, 2-3% by weight cholesterol and 1-2% by weight phospholipids other than phosphatidyl choline and phosphatidyl ethanolamine. The glycerides may include monoglycerides, diglycerides and triglycerides, however, is it preferable that triglycerides be present in a greater amount by weight than any other type of glyceride. It is particularly preferred that over 50% by weight of the glycerides are triglycerides.

The inventor has thus discovered that the essential ingredients to a particularly potent lipid membrane fluidizer consists essentially of neutral lipids and the phospholipids, phosphatidyl choline and phosphatidyl ethanolamine, and that a 7:3 by weight mixture of these the. neutral lipids and phospholipids, respectively, is particularly effective in fluidizing biological membranes.

The fluidity of biological membranes may be increased in vivo by oral, intravenous or topical administration. Alternatively, membrane fluidity may be increased in vitro by contacting a membrane with the composition of the present invention. Contacting biological membranes with the composition of the present invention results in reduction of the cholesterol levels of membranes, and may increase the phosphatidyl choline levels of membrances, leading to an increase in membrane fluidity. As demonstrated in the examples below, the special lipid mixture of the present invention substantially and rapidly increases the fluidity of biological membranes. Furthermore, the special mixture is relatively stable in vivo and is thus useful for effectively increasing membrane fluidity within an organism.

The composition of the present invention is suitable for in vivo use in mammals such as man for the treatment or prevention of conditions which are mediated by membrane lipid imbalance, conditions which are amenable to membrane lipid manipulation, or conditions which result from an increase in membrane microviscosity. As disclosed in commonly owned U.S. Patent No. 4,474,773 - (incorporated herein by reference), these conditions include:

a) various symptoms of aging and senescence (e.g., loss of mental functions and libido, increased vulnerabilityto bacterial contaminations, etc.);

b) dysfunctions of the immune system;

c) allergies;

d) mental disorders such as Bipolar Affective Disorder (manic-depression) and schizophrenia and the like;

e) mental retardation;

f) neurological disorders such as Alzheimer's disease, Parkinsonism, Tardive dyskinesia, Huntington's chorea, tremor, ataxia, epilepsy, and the like;

g) hyperlipidemic states such as hypertension, atherosclerosis, gallstones, cirrhosis and obesity, and the like;

h) withdrawal from alcohol and other drugs;

i) tolerance to alcohol and other drugs.

The composition is also useful for enhancing the immune function in normal elderly patients.

For the in vivo treatment of a biological membrane which is present in a mammal such as man, the composition of the present invention may be administered to the mammal in a pharmaceutically effective quantity sufficient to increase the lipid fluidity of biological membranes.

The composition of the present invention may be suspended in a pharmaceutically acceptable carrier such as saline, and administered to mammals parenterally, e.g., by intravenous injection or infusion, for the treatment or prevention of the above-mentioned conditions. The compositions may also be orally administered to mammals in pharmaceutically acceptable carriers such food materials (as a dietary supplement), as well as other forms of oral administration, as by means of tablets or capsules in known pharmaceutically acceptable carriers. When administering the composition of the present invention in the diet (using food as a carrier), it is preferable that the diet contains no other lipids in order to maximize the membrane fluidization capabilities of the composition. Examples of dietary supplements containg the composition include gelatin capsules and food bars. Additionally, the composition could be packaged seperately, for consumption after dissolving the composition in a non-lipid liquid.

The composition of the invention is also suitable for the treatment of dry skin, eczema and other skin disorders. For skin disorders, the composition of this invention may be administered topically in carriers such as creams, salves and the like,-or in a patch.

The fluidity of biological membranes can be increased in vitro by contacting the membranes with the composition of the present invention. Examples of in vitro membrane manipulations include treatment of sperm infertility, facilitation of tissue transplantations, and modulation of viral infectivity for use in vaccinations.

According to one embodiment, a composition according to the present invention consists of a pharmaceutically acceptable carrier and a 7:3 by weight mixture of neutral lipids and phospholipids respectively, the neutral lipids consisting essentially of glycerides and the phospholipids consisting essentially of phosphatidyl choline and phosphatidyl ethanolamine. The phospholipids are advantageously present in a ratio of about 2 parts by weight phosphatidyl choline and 1 part by weight phosphatidyl ethanolamine.

The present invention further relates to a method for preparing a composition, which composition is effective in extracting cholesterol from and increasing the membrane fluidity of biological membranes. The method comprises admixing about 7 parts by weight neutral lipids and about 3 parts by weight phospholipids, the neutral lipids consisting essentially of glycerides and the phospholipids consisting essentially of phosphatidyl choline and phosphatidyl ethanolamine. Advantageously, the phospholipids of the composition are in a ratio of about 2 parts by weight phosphatidyl choline and 1 part by weight phosphatidyl ethanolamine. Phosphatidyl choline, phosphatidyl ethanolamine and neutral lipids (essentially glycerides) may be purchased from commercial sources and admixed as set forth above. The neutral lipids may alternatively be prepared from natural sources (e.g., egg yolk) as, for example, shown in Example I below.

The invention is further illustrated by the following examples which are not intended to be limiting.

EXAMPLE I

Preparation of various mixtures of neutral lipids, phosphatidyl choline and phosphatidyl ethanolamine

Phosphatidyl choline (PC) and phosphatidyl ethanolamine (DE), both from hen egg yolk and of >99% purity, were purchased from Lipid Products - (Nutfield, England).

Neutral lipids (NL) from egg yolk were isolated as follows: One hen egg yolk (approximately 20 ml) was first mixed with 180 ml isopropanol and after 1 hour at room temperature was supplemented with 200 ml chloroform and mixed for another hour. The precipitate was removed by filtration and the total lipid extract was recovered by evaporating the supernatant. The lipid was then dissolved in 50 ml hot ethanol and mixed with 1 liter of 1 M Kcl in water in a separation funnel. The upper layer of neutral lipids was separated, washed twice with distilled water and then dried by lyophilization. Thin layer chromatography and chemical. analysis indicated over 90% glycerides (mostly triglycerides), 2-3% cholesterol and 1-2% phospholipids.

The 7:2:1 composition of the invention (7:2:1 mixture) was prepared by admixing 7 parts by weight of the prepared neutral lipids, 2 parts by weight of the purchased phosphatidyl choline and 1 part by weight of the purchased phosphatidyl ethanolamine.

Other mixture ratios of the above starting materials were prepared for comparative studies with the 7:2:1 mixture of the present invention. The results of these comparative studies are set forth in Examples which follow.

## EXAMPLE II

Comparisons of the effects of various lipid mixtures on membrane fluidity of human erythrocytes and lymphocytes

Human erythrocytes and lymphocytes were subjected to various lipid treatments to compare the effectiveness of the 7:2:1 mixture of the invention with other mixture ratios.

In all of the lipid treatments, polyvinylpyrrolidone (PVP, MW 40,000, Sigma) was used as the hydrophobic carrier. The PVP medium consisted of 3.5% PVP, 1% bovine serum albumin and 0.5% glucose in phosphate buffered saline pH 7.4 - (PBS). $^3$H-sodium borohydride (20 Ci/mmole) and $^3$H-cholesterol (40 Ci/mmole) were purchased from Amersham.

The lipids were introduced into the PVP medium by 1:100 dilution of an ethanol solution, while in the control PVP medium 1% ethanol was included.

Human erythrocytes and lymphocytes were isolated from freshly drawn heparinized blood by the standard Ficoll-Hypaque sedimentation technique (see, e.g., Boyum, A., Scan. J. Clin. Invest., 21:77-89 [1968]), and then washed twice with PBS. $5 \times 10^7$ erythrocytes or $3 \times 10^6$ lymphocytes per ml PVP medium containing 0.5 mg/ml lipid were incubated at 37° with gentle shaking in loosely capped glass vials. The treated cells were washed once with PBS then with 0.25 M CsBr and once again with PBS. The wash with 0.25 M CsBr was found to remove traces of lipids adhered to the cell surface as verified with lipid mixtures containing $^3$H-NL, $^3$H-PC and $^3$H-PE.

For labelling of cells with $^3$H-cholesterol incubation with PVP medium containing 10 g/ml, $^3$H-cholesterol was carried out under the above conditions, and was allowed to proceed until an increase of about 1% in membrane cholesterol was reached.

The standard technique of steady-state fluorescence depolarization with 1,6-diphenyl 1,3,5-hexatriene (DPH) as a probe, was used to measure membrane fluidity (see, e.g., Shinitzky, M. and Barenholz Y., Biochim. Biophys. Acta, 515:367-394 [1978], and Shinitzky, M. and Yuli, I., Chem. Phys. Lipids, 30:261-282 [1982]).

Erythrocyte membranes at a final dilution of 1:200 or intact lymphocytes ($2 \times 10^6$ per ml) in PBS were labelled with DPH and the degree of fluorescence polarization, P, was determined as previously described in Shinitzky, M. and Inbar, M., Biochim. Biophys. Acta, 433:133-149 [1976]. The empirical linear scale of $2P/(0.46-P)$ was used as an approximate presentation of the membrane lipid microviscosity.

The impermeable reagent 2,4,6-trinitrobenzene sulfonic acid (TNBS) was used for measuring the availability of PE at the outer surface of the lipid assemblies. Lipid mixtures (0.5 mg/ml) in PBS were sonicated for 5 minutes in the absence and in the presence of 0.1% Triton X-100. This detergent was applied for disintegration of the lipid assemblies to form permeable mixed micelles. Into 1 ml of each of the mixtures 10 l of 100 mg/ml TNBS - (color-free sodium salt from Research Organics Inc.) in water was added. In the other 1 ml samples, which served as reference, 10 l of water was added. After 2 hours incubation at room temperature, the optical density at 420 nm of the TNBS labelled samples, in the absence ($O.D_{PBS}$) and in the presence of Triton X-100 ($O.D_{triton}$), were measured versus their reference samples in triplicates. The ratio $O.D._{PBS}/O.D._{triton}$ was taken as the fraction of PE available for TNBS labelling.

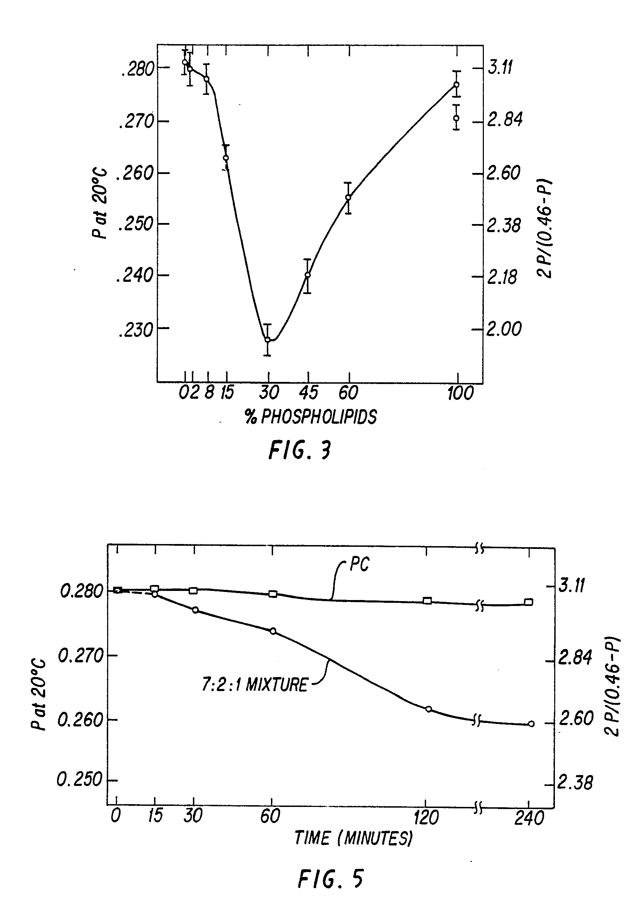

The membrane fluidization potency of the various NL-PC-PE mixtures was tested on human erythrocytes and lymphocytes. The mixtures were all of 0.5 mg/ml and consisted of the phospholipids PC and PE (2/1) and increasing amounts of NL. Results obtained with these two cell types are shown in Figs. 2 and 3.

Fig. 2 shows the fluidization of human erythrocyte membranes ($5 \cdot 10^7$ cells/ml) with various NL-PC-PE mixtures (0.5 mg/ml) after 18 hours incubation at 35°. Results are presented as the mean S.D. of the degree of DPH fluorescence polarization, P, obtained for membrane samples of 10 humans. Results obtained with PC alone under the same conditions ( ) are included for comparison.

Fig. 3 shows the fluidization of human lymphocyte membranes ($3 \cdot 10^6$ cell/ml) with NL-PC-PE mixtures or with PC ( ) (0.5 mg/ml) after 3 hours incubation at 37°. Results are presented as in Fig. 2.

As shown in Figs. 2 and 3, the membrane fluidization profile in both cells is remarkably similar and indicates that the mixture of NL-PC-PE 7:2:1 (mixture 721) is the most potent membrane fluidizer.

## EXAMPLE III

Comparisons of the effects of various lipid mixtures on membrane fluidity of mouse thymocytes

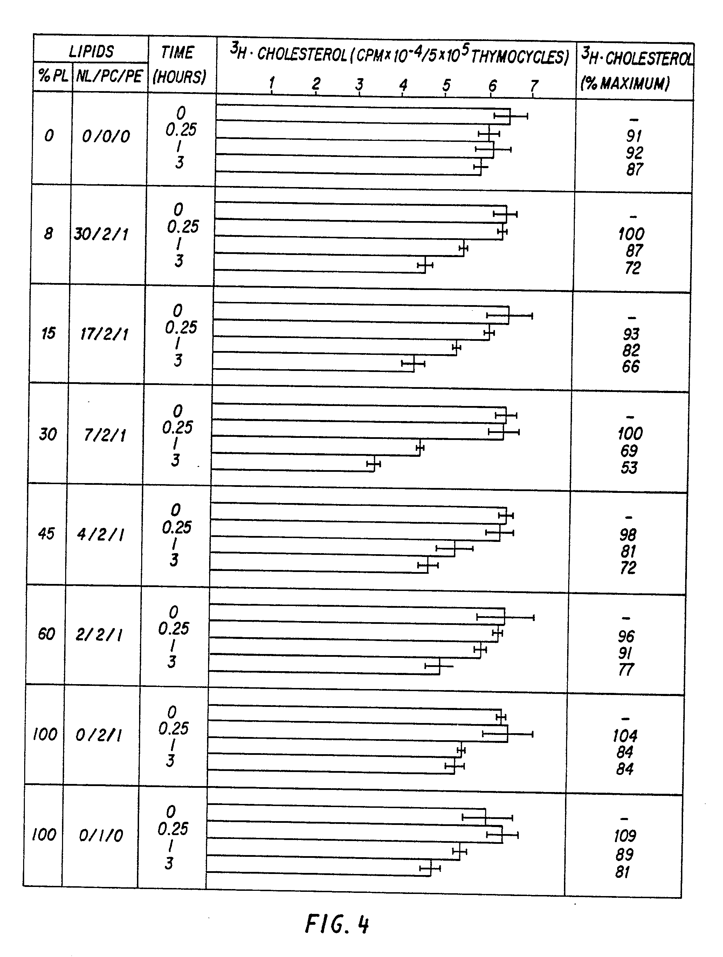

Various lipid compositions and phosphatidyl choline alone were compared to the 7:2:1 mixture prepared in Example I as to their effect on the rate of extraction of cholesterol from the plasma membranes of mouse thymocytes. $^3$H-cholesterol was incorporated into the cell plasma membranes by exchange with the external medium. The rate of reduction in radioactive counts upon incubation with lipid containing medium was determined. The results are shown in Fig. 4.

In the control experiment (no lipid treatment- upper panel of Fig. 4), the rate of reduction in ³H- cholesterol represents the rate of exchange with the control medium which should be taken as the baseline. The results are mean SD of triplicates.

Fig. 4 clearly demonstrates that the 7:2:1 mixture of the present invention is most effective in the rate of extraction of excess cholesterol from the cell membranes of the mixtures tested.

## EXAMPLE IV

Membrane fluidization effects of the 7:2:1 mixture of the invention compared to Phosphatidyl Choline alone

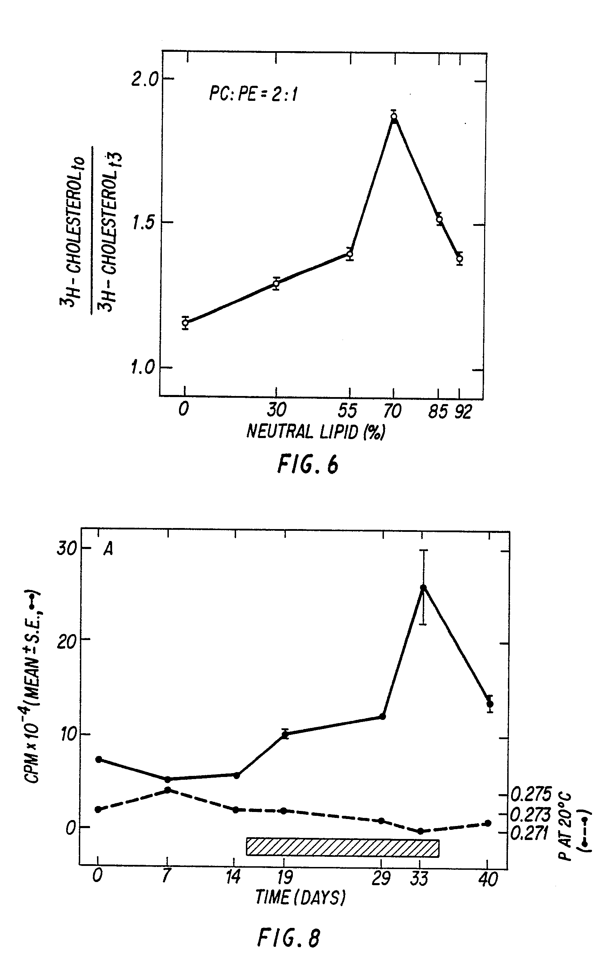

In a study similar to Example II, the fluidization effects of 7:2:1 mixture was compared to phosphatidyl choline alone using human lymphocytes. Fig. 5 shows the increase in fluidization of human lymphocytes incubated at room temperature with mixture 7:2:1 (0.5 mg/ml) for different periods of time relative to PC. Results obtained with control medium are also included.

As shown in Fig. 5, the 7:2:1 mixture mediates its effect within a relatively short period of incubation even at room temperature, and is markedly more potent than PC alone.

## EXAMPLE V

Cholesterol removal by various mixtures of lipids

In an experiment similar to Example III, human peripheral blood lymphocytes with ³H-cholesterol in their membranes were incubated at 37°C with various NL-PC-PE mixtures. 2:1 Mixtures of PC-PE were mixed with increasing amounts of NL and compared in the test. The radioactivity per cell population was measured after 0 and 3 hour incubation times and the results are presented as the ratio of counts per minute at those times.

As shown in Fig. 6, the 7:2:1 mixture is the most potent for cholesterol extraction compared to all other mixtures tested.

## EXAMPLE VI

The effect of 7:2:1 diet compared to standard diet in rats

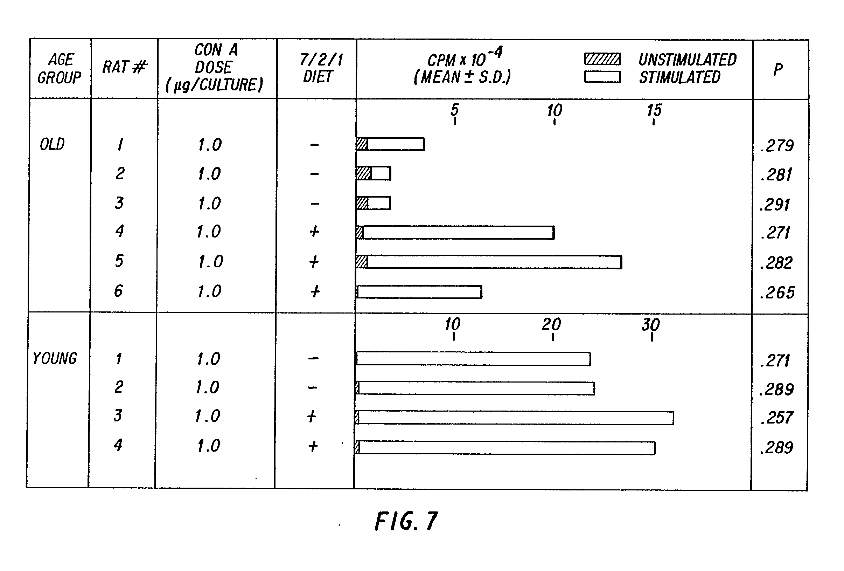

The effect of 7:2:1 diet on immune competence of young (3-5 months) and old (22-24 months) rats was examined in a controlled study. The diet consisted of Purina™ chow plus or minus 4-6% by weight 7:2:1 mixture over a period of one month. Mitogenic stimulations of splenocytes were determined for each individual animal with Con A. Splenocytes from the animals were tested in vitro for mitogenic response by Con A stimulation of ³H- thymidine incorporation. The results are presented in Fig. 7 for each individual rat as means S.D. of triplicates.

As indicated in Fig. 7, splenocytes from old rats on a standard diet are considerably less responsive to mitogenic stimulation than splenocytes from young rats. 7:2:1 Diet in the old increased the splenocyte responsiveness by about 5-fold to a level similar to that found in the young. Splenocytes from young were only slightly activated by the 7:2:1 diet. Similar results were also obtained with lymph node cells from mice and rats.

The overall restoration of immune competence by 7:2:1 diet was examined by increase in survival of old animals infected with pathogenic bacteria. The average ability of the old animals to cope with the infectious disease was increased markedly by 7:2:1 diet and reached a level similar to that of the young animals.

## EXAMPLE VII

Immunological study with humans involving 7:2:1 supplemented diet

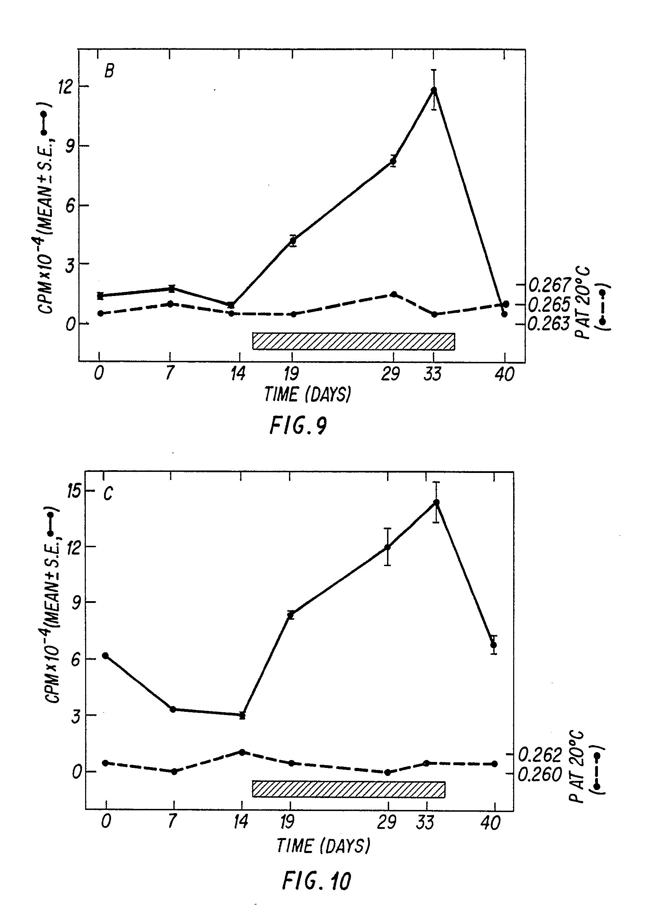

This immunological study involved participants over 75 years of age who were immune suppressed but did not display any organic disease and were not taking immunosuppressive drugs. The mitogenic responsiveness of peripheral blood lymphocytes was tested in each of the participants at least 3 times within 3 weeks before entering the study in order to well define their basal imune competence. A 7:2:1 mixture supplement of 10-15 gr was given each morning during a period of several weeks and the responsiveness of peripheral blood lymphocytes to mitogens was tested every several days. The 7:2:1 diet was then stopped and the effect on mitogenic stimulation was measured 7 days later. Typical results obtained in 3 subjects are shown in Figs. 8, 9 and 10 which illustrate the responsiveness of peripheral blood lymphocytes to PHS stimulation before, during (......) and after 7:2:1 diet in the subjects. As shown in Figs. 8, 9 and 10, after several days of 7:2:1 diet, a significant increase in response to mitogens was observed. After about 3 weeks it reached a level typical to that found in the young. Upon cessation of the diet, the lymphocyte responsiveness declined towards the initial basal level.

## Claims

1. A composition consisting essentially of about seven parts by weight neutral lipids, and about three parts by weight phospholipids, wherein said neutral lipids consist essentially of glycerides and said phospholipids consist essentially of phosphatidyl choline and phosphatidyl ethanolamine.

2. A composition according to claim 1, wherein said phospholipids are present in a ratio of about 2 parts by weight phosphatidyl choline and about 1 part by weight phosphatidyl ethanolamine.

3. A composition according to claim 1 or claim 2, wherein said phospholipids and said neutral lipids are derived from natural sources.

4. A composition according to claim 3, wherein said natural sources are egg yolk, soy beans or mixtures thereof.

5. A composition according to any one of claims 1 to 4, wherein said glycerides comprise triglycerides.

6. A composition according to any one of claims 1 to 5, wherein, of said glycerides, triglycerides are present in a greater amount than any other type of glyceride.

7. A composition according to claim 6, wherein over 50% by weight of said glycerides are triglycerides.

8. A composition according to any one of claims 1 to 7, wherein said neutral lipids comprise a fraction of egg yolk, and said fraction comprises about 90% by weight glycerides, from about 2% to about 3% by weight cholesterol, and from about 1% to about 2% by weight phopholipids.

9. A composition according to any one of claims 1 to 8 adapted for oral administration.

10. A composition adapted for parenteral or topical administration comprising a composition according to any one of claims 1 to 8 in admixture with a pharmaceutically acceptable carrier.

11. A composition according to claim 1 or claim 2, consisting of a pharmaceutically acceptable carrier and about seven parts by weight neutral lipids and about three parts by weight phospholipids, the phospholipids consisting essentially of phosphatidyl choline and phosphatidyl ethanolamine, and said neutral lipids consisting essentially of glycerides.

12. A composition according to any one of claims 1 to 11, for use for increasing the fluidity of a biological membrane present in a warm-blooded mammal, for example, for the treatment or prevention of conditions which are mediated by membrane lipid imblance, conditions which are amenable to membrane lipid manipulation, or conditions which result from an increase in membrane micro-viscosity, such as:

a) various symptoms of aging and senescence (e.g., loss of mental functions and libido, increased vulnerability of bacterial contaminations, etc.);

b) dysfunctions of the immune system;

c) allergies;

d) mental disorders such as Bipolar Affective Disorder (manic-depression) and schizophrenia and the like;

e) mental retardation;

f) neurological disorders such as Alzheimer's disease, Parkinsonism, Tardive dyskinesia, Huntington's chorea, tremor, ataxia, epilepsy, and the like;

g) hyperlipidemic states such as hypertension, atherosclerosis, gallstones, cirrhosis and obesity, and the like;

h) withdrawal from alcohol and other drugs;

i) tolerance to alcohol and other drugs; or

j) skin disorders such as eczema, dry skin and the like.

13. A method for increasing the fluidity of a biological membrane in vitro comprising contacting the membrane with a composition according to any one of claims 1 to 12.

14. A method for reducing cholesterol content of a biological membrane comprising contacting the membrane in vitro with a composition according to any one of claims 1 to 12.

15. A method according to claim 13 or claim 14 wherein said biological membrane is contacted with said composition to treat sperm infertility, facilitate tissue transplantations, or modulate viral infectivity for use in vaccinations.

16. A method for preparing a composition which is effective in increasing the fluidity of biological membranes, the method comprising admixing about seven parts by weight neutral lipids and about three parts by weight phospholipids, the phospholipids consisting essential of phosphatidyl choline and phosphatidyl ethanolamine, and the neutral lipids consisting essentially of glycerides.

17. A method according to claim 16, comprising admixing about seven parts by weight neutral lipids, about two parts by weight phosphatidyl choline and about one part by weight phosphatidyl ethanolamine, said neutral lipids consisting essentially of glycerides.

BATCHELLOR. KIRK & EYLES

CHARTERED PATENT AGENTS
EUROPEAN PATENT ATTORNEYS
TRADE MARK AGENTS

G T KIRK B Sc (Eng) A.M.I E.E. C.P.A
C T EYLES. M A.. Ph.D. (Cantab) C.Chem M R.S C.. C.P.A
N SHINDLER. B Sc (Eng) A.C.G.I. C.P A

N J EYLES. B.Sc. PH.D. (Cantab) C Chem M R.S C. C.P A
R BUTTRICK. B Sc (Eng & Phys) C P A

2 PEAR TREE COURT
FARRINGDON ROAD
LONDON EC1R ODS

TELEPHONE: 01-253 1563
TELEX 8952160 BKEPAT G
CABLE ADDRESS· BAKIRCO. LONDON E C 1

YOUR REF

OUR REF   CTE/JFA/86151

13th October, 1986

REGISTERED

European Patent Office,
Receiving Section,
P.B. 5818 Patentlaan 2,
2280 HV Rijswijk (ZH),
HOLLAND.

Dear Sirs,

European Patent Application No. 86305736.0
Yeda Research and Development Company Limited

With reference to the EPO Form 1121 mailed to me on 5th September 1986 I lodge herewith an authorisation.

With reference to the EPO Form 1150 mailed to me on 5th September 1986 I lodge herewith in triplicate a retyped abstract. Also accompanying this letter are fresh copies of pages 1 to 17 of the specification to replace the copies originally filed, the legibility of which has suffered as these were photocopies of the text which had been transmitted to us by facsimile. I believe that the new copies of pages 1 to 17 are identical to the original copies of pages 1 to 17 with the sole exception of the insertion at page 17, line 20 of the symbol in parenthesis in further explanation of Figures 8, 9 and 10.

Formal drawings were forwarded to you with my letter of 6th October 1986.

Yours faithfully,

EYLES, Christopher Thomas.

**FIG. 1**

**FIG.2**

FIG. 3

FIG. 5

| LIPIDS | | TIME | $^3$H · CHOLESTEROL (CPM×10$^{-4}$/5×10$^5$ THYMOCYCLES) | $^3$H · CHOLESTEROL |
|---|---|---|---|---|
| % PL | NL/PC/PE | (HOURS) | 1  2  3  4  5  6  7 | (% MAXIMUM) |
| 0 | 0/0/0 | 0<br>0.25<br>1<br>3 | | —<br>91<br>92<br>87 |
| 8 | 30/2/1 | 0<br>0.25<br>1<br>3 | | —<br>100<br>87<br>72 |
| 15 | 17/2/1 | 0<br>0.25<br>1<br>3 | | —<br>93<br>82<br>66 |
| 30 | 7/2/1 | 0<br>0.25<br>1<br>3 | | —<br>100<br>69<br>53 |
| 45 | 4/2/1 | 0<br>0.25<br>1<br>3 | | —<br>98<br>81<br>72 |
| 60 | 2/2/1 | 0<br>0.25<br>1<br>3 | | —<br>96<br>91<br>77 |
| 100 | 0/2/1 | 0<br>0.25<br>1<br>3 | | —<br>104<br>84<br>84 |
| 100 | 0/1/0 | 0<br>0.25<br>1<br>3 | | —<br>109<br>89<br>81 |

FIG. 4

0 213 724

FIG. 6

FIG. 8

| AGE GROUP | RAT # | CON A DOSE ($\mu$g/CULTURE) | 7/2/1 DIET | CPM x $10^{-4}$ (MEAN $\pm$ S.D.) | | | | P |
|---|---|---|---|---|---|---|---|---|
| | | | | 5 | 10 | 15 | | |
| OLD | 1 | 1.0 | – | | | | | .279 |
| | 2 | 1.0 | – | | | | | .281 |
| | 3 | 1.0 | – | | | | | .291 |
| | 4 | 1.0 | + | | | | | .271 |
| | 5 | 1.0 | + | | | | | .282 |
| | 6 | 1.0 | + | | | | | .265 |
| | | | | 10 | 20 | 30 | | |
| YOUNG | 1 | 1.0 | – | | | | | .271 |
| | 2 | 1.0 | – | | | | | .289 |
| | 3 | 1.0 | + | | | | | .257 |
| | 4 | 1.0 | + | | | | | .289 |

Legend: ▨▨▨ UNSTIMULATED   ▭ STIMULATED

FIG. 7

0 213 724

FIG.9

FIG.10

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP-A-0 074 251 (YEDA RESEARCH AND DEVELOPMENT CO. LTD.) * Pages 27-30, claims 1-18 * | 1-17 | A 61 K 31/685// (A 61 K 31/685 A 61 K 31:23 ) |
| X | GB-A-2 080 324 (KURT KAMPFFMEYER MÜHLENVEREINIGUNG KG) * Page 1, lines 99-121, claims 1-6 * | 1-17 | |
| X | EP-A-0 148 303 (A. VON MIETZKO) * Page 7, line 1 - page 8, line 20, claims 1-9 * | 1-17 | |
| X | FR-A-2 455 888 (THE GREEN CROSS CORP.) * Page 11, lines 1-10, claim 1 * | 1-17 | |
| P,X | EP-A-0 180 786 (E. PISTOLESI) * Pages 10,11, claims 1-10 * | 1-17 | TECHNICAL FIELDS SEARCHED (Int. Cl.4) A 61 K |
| A | DE-A-2 556 592 (A. NATTERMANN & CIE GmbH) | 1-17 | |
| A | EP-A-0 051 833 (EXTRAKTA STRAUSS GmbH) | 1-17 | |

The present search report has been drawn up for all claims

| Place of search | Date of completion of the search | Examiner |
|---|---|---|
| THE HAGUE | 03-11-1986 | BRINKMANN C. |